# EUROPEAN PATENT APPLICATION

(11) **EP 4 279 582 A1**
(43) Date of publication of application: **22.11.2023**
(21) Application number: 22739495.4
(22) Date of filing: 14.01.2022
(51) Int. Cl.: C12N 5/071

(54) **METHOD FOR CULTURING HUMAN HEPATIC PARENCHYMAL CELL, CULTURED HUMAN HEPATIC PARENCHYMAL CELL, CULTURE MEDIUM AND METHOD FOR PRODUCING SAME, AND METHOD FOR PRODUCING CULTURE SUPERNATANT OF HUMAN HEPATIC NONPARENCHYMAL CELL**

(30) Priority: 14.01.2021 JP 2021004357
(71) Applicant: JSR Corporation, Tokyo 105-8640 (JP); Keio University, Tokyo, 108-8345 (JP)
(72) Inventor: MASUDA, Norio, Tokyo 105-8640 (JP); SATO, Toshiro, Tokyo 160-8582 (JP)
(74) Representative: TBK
(86) International application number: PCT/JP2022/001162
(87) International publication number: WO 2022/154090

(57) **Abstract**

Provided is a method of culturing a human hepatic parenchymal cell, including culturing a human hepatic parenchymal cell in a medium containing: a culture supernatant of a human hepatic nonparenchymal cell; a Wnt signaling promoter; and a mitogenic growth factor.

## Description

### Field of the Invention

The present invention relates to a method of culturing a human hepatic parenchymal cell, a cultured human hepatic parenchymal cell obtained by the method, a medium to be used in the method and a method of producing the same, and to a method of producing a culture supernatant of a human hepatic nonparenchymal cell.

### Background Art

In pharmacokinetic tests for pharmaceutical development, an *in vivo* test using a rodent and an *in vitro* test using rodent-derived hepatocytes (hepatic parenchymal cells) are performed. However, a test with a rodent or cells thereof entails a species difference, and hence it is difficult to predict toxicity that occurs in a human-specific manner. Meanwhile, primary human hepatocytes are difficult to stably obtain in an amount required for a pharmacokinetic test for pharmaceutical development. Accordingly, there is a demand that a hepatocyte line (master cell line) excellent in growth properties, which is capable of long-term culture, be established from primary human hepatocytes.

A liver organoid is a hepatocyte culture product cultured from hepatocytes, and is promising as a stable hepatocyte supply source for pharmaceutical development. An improved method of constructing a liver organoid has been developed. In Patent Literature 1, there is a disclosure of a method of producing a human liver organoid involving using a medium containing an epidermal growth factor (EGF), a Notch inhibitor, and a transforming growth factor-β (TGF-β) inhibitor, or a medium containing EGF, a BMP inhibitor, and a Wnt agonist.

### Citation List

### Patent Literature

[Patent document 1] US 2018/0066233 A1

### Summary of Invention

### Technical Problem

The present invention provides a method of culturing a human hepatic parenchymal cell excellent in growth properties, which is capable of long-term culture, a cultured human hepatic parenchymal cell produced by the method, and a medium to be used in the method. The present invention also provides a method of producing a culture supernatant of a human hepatic nonparenchymal cell.

### Solution to Problem

The present invention includes the following embodiments.
[1] A method of culturing a human hepatic parenchymal cell, comprising culturing a human hepatic parenchymal cell in a medium containing: a culture supernatant of a human hepatic nonparenchymal cell; a Wnt signaling promoter; and a mitogenic growth factor.
[2] The method according to Item [1], wherein the Wnt signaling promoter contains a Wnt family member protein.
[3] The method according to Item [2], wherein the Wnt signaling promoter further contains an R-spondin super family protein.
[4] The method according to any one of Items [1] to [3], wherein the mitogenic growth factor contains one or more kinds selected from the group consisting of: an epidermal growth factor (EGF); a fibroblast growth factor (FGF); and a hepatocyte growth factor (HGF).
[5] The method according to any one of Items [1] to [4], wherein the medium further contains one or more kinds selected from: a Rho-kinase (ROCK) signaling inhibitor; a transforming growth factor-β (TGF-β) signaling inhibitor; and an interleukin-6 (IL-6) family cytokine.
[6] The method according to Item [5], wherein the medium contains the ROCK signaling inhibitor.
[7] The method according to Item [5] or [6], wherein the medium contains the TGF-β signaling inhibitor.
[8] The method according to any one of Items [5] to [7], wherein the medium contains the IL-6 family cytokine.
[9] The method according to any one of Items [1] to [8], wherein the medium further contains one or more kinds selected from: retinoic acid; nicotinamide; a cAMP activator; gastrin; a neurobiological supplement; and an antioxidant.
[10] The method according to any one of Items [1] to [9], wherein the culture supernatant of a human hepatic nonparenchymal cell is produced by: culturing a human hepatic nonparenchymal cell in a medium for human hepatic nonparenchymal cell culture, the medium containing a Wnt signaling promoter and a mitogenic growth factor; and separating a supernatant from the medium after the culturing.
[11] The method according to Item [10], wherein the medium for human hepatic nonparenchymal cell culture further contains a ROCK signaling inhibitor.
[12] The method according to Item [10] or [11], wherein the medium for human hepatic nonparenchymal cell culture further contains one or more kinds selected from: an IL-6 family cytokine; a TGF-β signaling inhibitor; retinoic acid; nicotinamide; a cAMP activator; gastrin; a neurobiological supplement; and an antioxidant.
[13] A cultured human hepatic parenchymal cell, which has been cultured by the method of any one of Items [1] to [12].
[14] A medium, comprising: a culture supernatant of a human hepatic nonparenchymal cell; a Wnt signaling promoter; and a mitogenic growth factor.
[15] A method of producing a culture supernatant of a human hepatic nonparenchymal cell, the method comprising: culturing a human hepatic nonparenchymal cell in a medium containing a Wnt signaling promoter and a mitogenic growth factor; and separating a supernatant from the medium after the culturing.
[16] A method of producing a medium, comprising mixing a culture supernatant of a human hepatic nonparenchymal cell produced by the method of Item [15], a Wnt signaling promoter, and a mitogenic growth factor.

### Advantageous Effects of Invention

The present invention provides a method of culturing a human hepatic parenchymal cell excellent in growth properties, which is capable of long-term culture. According to the present invention, human hepatic parenchymal cells useful for pharmaceutical development and the like can be stably supplied.

### Brief Description of Drawings

FIG. 1 shows the growth properties of human hepatic parenchymal cells of Experimental Example 2 to Experimental Example 4.
FIG. 2-1 shows activated pathways in cultured human hepatic parenchymal cells of Experimental Example 2 and Experimental Example 4.
FIG. 2-2 is a continuation of FIG. 2-1.

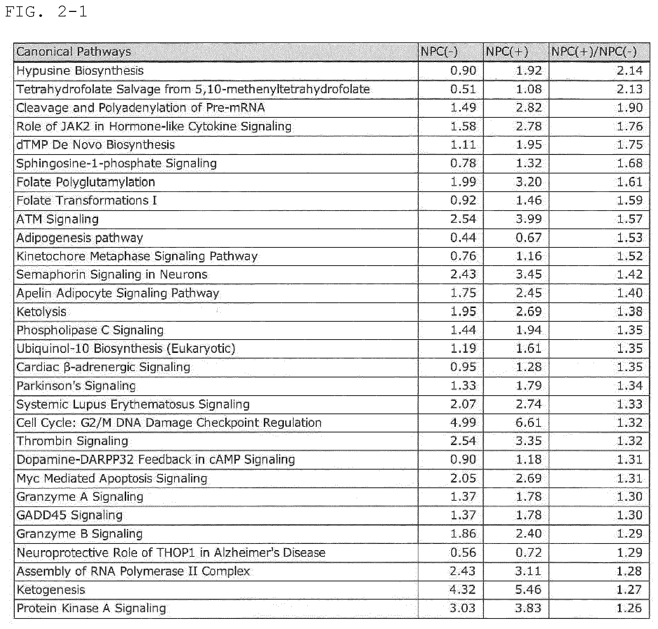

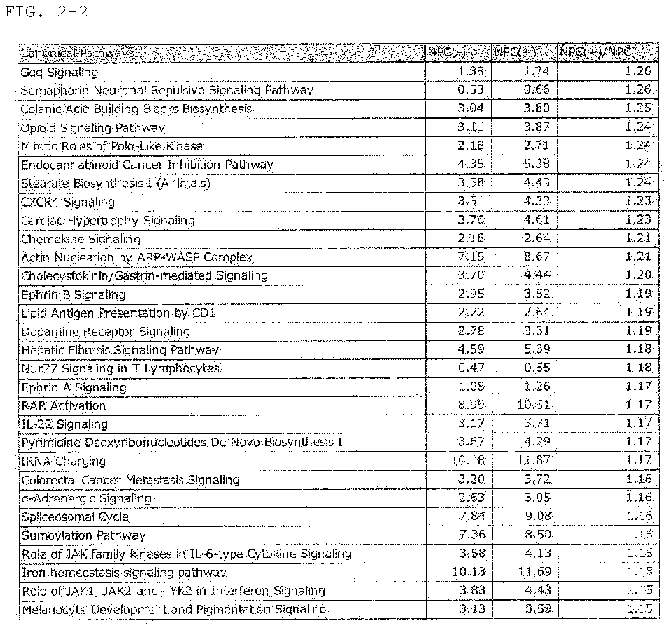

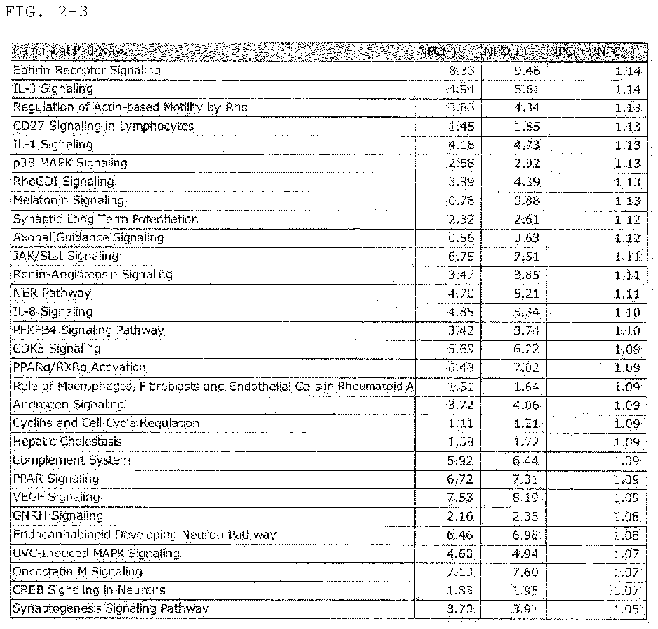

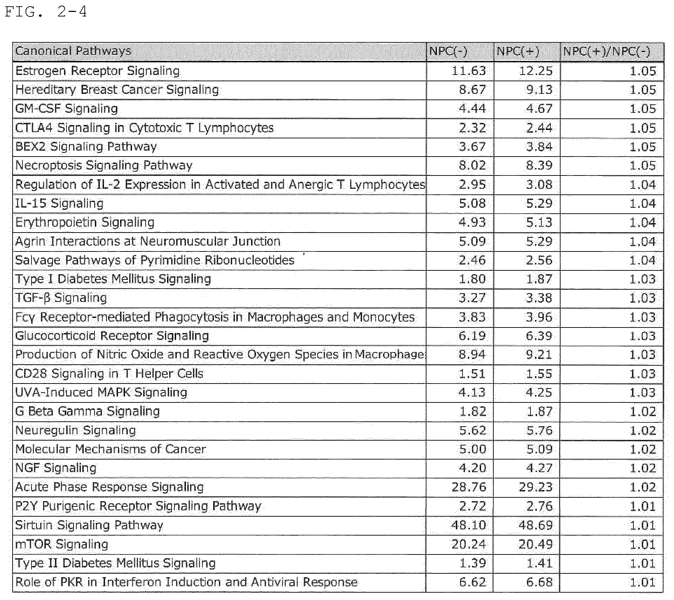

FIG. 2-3 is a continuation of FIG. 2-2.
FIG. 2-4 is a continuation of FIG. 2-3.

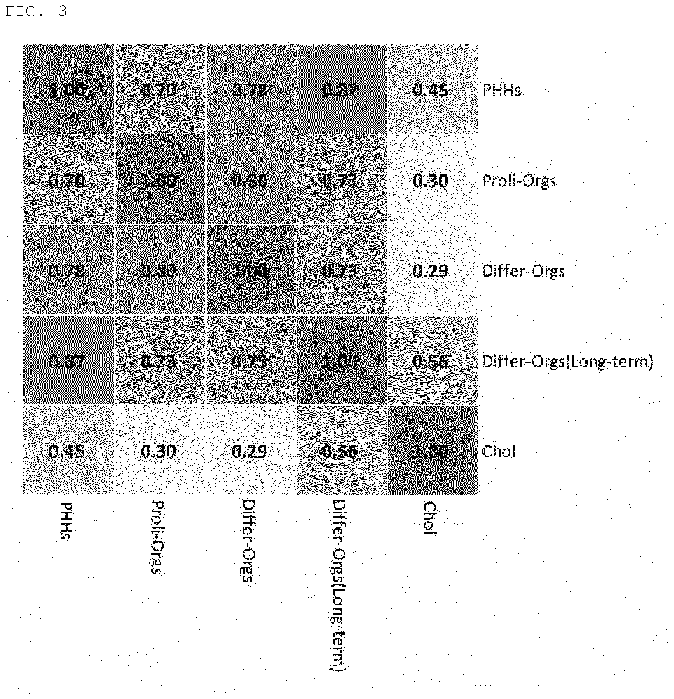

FIG. 3 shows correlations in gene expression among primary hepatic parenchymal cells (primary human hepatocyes, PHHs), human hepatic parenchymal cells (Proli-Orgs) of Experimental Example 2, mature human hepatic parenchymal cells (Differ-Orgs and Differ-Orgs (long-term)) of Experimental Example 6, and human cholangiocytes (Chol) of Experimental Example 7. Numerical values in the figure each represent a correlation coefficient.

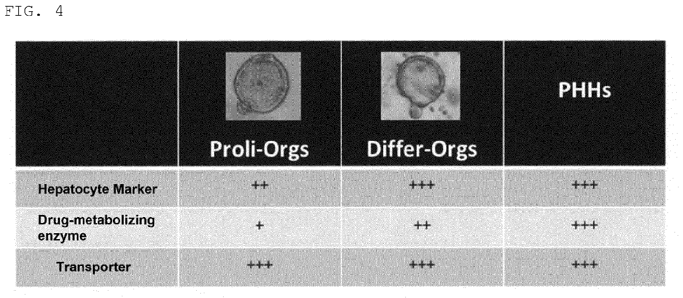

FIG. 4 shows microscopic images and gene expression amounts of the PHHs, the Proli-Orgs, and the Differ-Orgs (long-term).

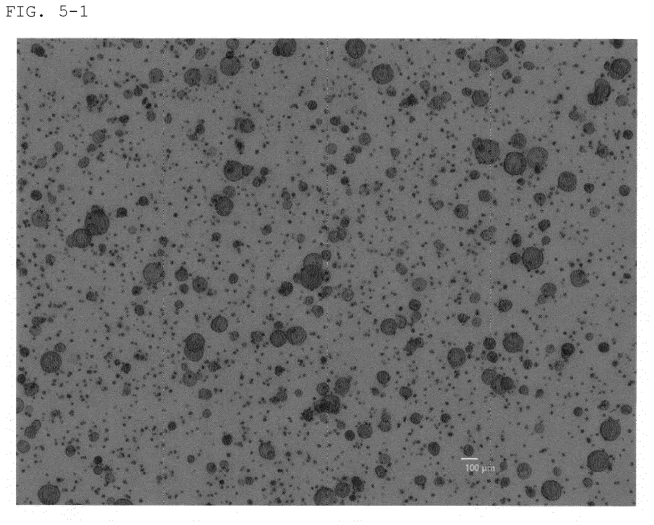

FIG. 5-1 is a microscopic image of cultured human hepatic parenchymal cells of Experimental Example 2.

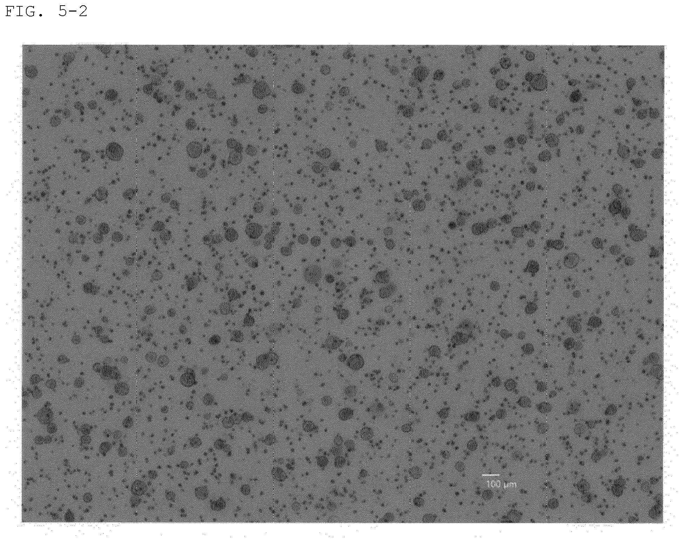

FIG. 5-2 is a microscopic image of cultured human hepatic parenchymal cells of Experimental Example 9.

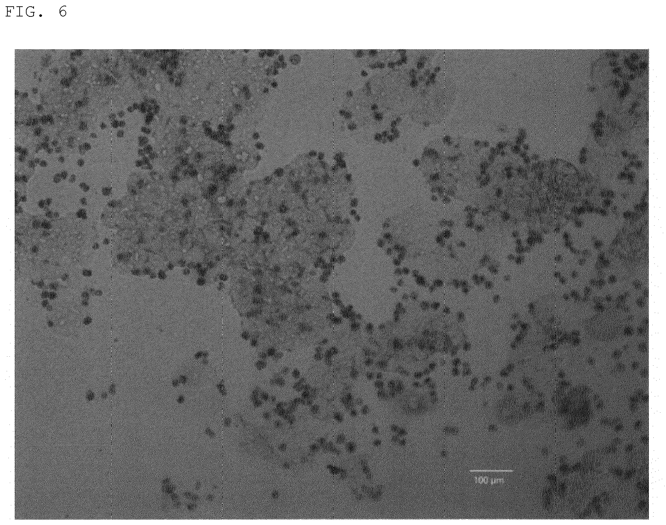

FIG. 6 is a microscopic image of cultured human hepatic parenchymal cells of Experimental Example 10.

### Description of Embodiments

Now, the present invention is described in detail by way of embodiments. However, the present invention is not limited to the following embodiments.

Components given as examples herein may be used alone or in combination thereof, unless otherwise stated.

Herein, an expression representing a numerical range such as "from A to B" has the same meaning as "A or more and B or less," and includes A and B in the numerical range.

With regard to a protein given as an example herein, even a mutant protein having a substitution, a duplication, a deletion, an insertion, or a frame shift in an amino acid sequence thereof or a base sequence coding therefor is included in the protein before the mutation when the mutant protein has a function equivalent to that before the mutation.

Herein, the expression "medium containing a substance X" or "in the presence of a substance X" means a medium supplemented with an exogenous substance X, a medium containing an exogenous substance X, or in the presence of an exogenous substance X. That is, when cells or a tissue present in the medium endogenously expresses, secretes, or produces the substance X, the endogenous substance X is distinguished from the exogenous substance X, and a medium free of the exogenous substance X is understood to fall outside the category of the "medium containing a substance X," even when containing the endogenous substance X.

### <Method of culturing Human Hepatic Parenchymal Cell>

According to one embodiment of the present invention, there is provided a method of culturing a human hepatic parenchymal cell. The culture method according to this embodiment comprises culturing a human hepatic parenchymal cell in a medium containing: a culture supernatant of a human hepatic nonparenchymal cell; a Wnt signaling promoter; and a mitogenic growth factor (hereinafter sometimes referred to as "step 1"). By the culture method according to this embodiment, a cultured human hepatic parenchymal cell according to one embodiment of the present invention is produced.

The liver is made up of hepatic parenchymal cells (or hepatocytes), which are responsible for the essence of liver function, and hepatic nonparenchymal cells, which support the growth and survival of the hepatic parenchymal cells. Examples of the hepatic nonparenchymal cells include hepatic stellate cells, sinusoidal endothelial cells, and Kupffer cells. A population of hepatic parenchymal cells generally includes hepatic stem cells, preferably epithelial hepatic stem cells.

In this embodiment, the human hepatic parenchymal cell to be subjected to the culture in the step 1 may be isolated from a liver fragment, which is collected from a human individual, by a known method such as a collagenase perfusion method, or a primary human hepatic parenchymal cell or a passaged human hepatic parenchymal cell may be prepared from an isolated human hepatic parenchymal cell and used as the human hepatic parenchymal cell to be subjected to the culture in the step 1. Alternatively, a commercially available primary human hepatic parenchymal cell or passaged human hepatic parenchymal cell may be used as the human hepatic parenchymal cell to be subjected to the culture in the step 1. The human hepatic parenchymal cell to be subjected to the culture in the step 1 is preferably an isolated human hepatic parenchymal cell substantially free of a human hepatic nonparenchymal cell, more preferably a primary human hepatic parenchymal cell and a passaged human hepatic parenchymal cell, still more preferably a primary human hepatic parenchymal cell.

As a method for the culture of the human hepatic parenchymal cell in the step 1, there are given, for example, suspension culture and adherent culture. The "suspension culture" is a method involving culturing cells, which are brought into contact with an extracellular matrix (ECM) as required, while maintaining a state of being suspended in a medium without being caused to adhere to the surface of a culture vessel. The "adherent culture" is a method involving culturing cells in a state of being caused to adhere to the surface of a culture vessel via an ECM or the like as required.

At the time of the culture, the human hepatic parenchymal cell is preferably cultured while being brought into contact with an extracellular matrix (ECM). The human hepatic parenchymal cell may be brought into contact with the ECM by, for example, embedding the human hepatic parenchymal cell in the ECM, adding the human hepatic parenchymal cell to a culture vessel coated with the ECM, or adding the human hepatic parenchymal cell from a donor to a medium having the ECM suspended therein. In a preferred embodiment, an ECM precursor is brought into contact with the human hepatic parenchymal cell in the process of being gelled, to thereby prepare a human hepatic parenchymal cell embedded in the ECM, which is then cultured.

Examples of the ECM or the ECM precursor (hereinafter referred to as "ECM etc.") include: components in basement membranes, such as Type IV collagen, laminin, heparan sulfate proteoglycan, and entactin; and glycoproteins each present in an intercellular gap, such as collagen, laminin, entactin, fibronectin, and heparin sulfate. Such ECM etc. may be prepared in accordance with a conventional method, or a commercially available product may be used. Examples of the commercially available ECM etc. for cell culture include Matrigel (trademark) (name of a product of Corning Incorporated) and Human Laminin (name of a product of Sigma-Aldrich).

The culture vessel to be used for the suspension culture is preferably a culture vessel having a non-cell-adherent surface. Examples of the culture vessel having a non-cell-adherent surface include: a vessel having a surface treated with a non-cell-adherent material such as an MPC polymer; and a vessel having a surface shaped with irregularities. The culture vessel to be used for the adherent culture is preferably a culture vessel having a surface coated with an ECM.

The culture in the step 1 is performed under the condition of generally from 30°C to 50°C, preferably from 32°C to 48°C, more preferably from 34°C to 46°C. In addition, the culture in the step 1 is performed under an atmosphere having a carbon dioxide content of generally from 1 vol% to 15 vol%, preferably from 2 vol% to 14 vol%, more preferably from 3 vol% to 13 vol%. The above-mentioned culture conditions may be achieved by adjusting the temperature and carbon dioxide content of a container (e.g., an incubator) in which the culture vessel is placed.

A component contained in the medium is deactivated or disappears during the culture, and hence the medium is generally changed every 1 day to 5 days. In addition, the human hepatic parenchymal cell obtained by the culture may be subjected to passage culture by being taken out of the medium, subjected to dispersion treatment as required, and then cultured again under similar conditions.

In the culture in this embodiment, the human hepatic parenchymal cell has excellent growth properties, and can be cultured for at least 30 days, preferably at least 90 days, enabling long-term culture of human hepatic parenchymal cells.

### <Medium for Culture of Human Hepatic Parenchymal Cell>

The medium to be used in the step 1 is a medium containing a culture supernatant of a human hepatic nonparenchymal cell, a Wnt signaling promoter, and a mitogenic growth factor. The medium to be used in the step 1 is hereinafter sometimes referred to as "medium 1" .

As shown in the results of RNA-seq in Examples to be described later, the culture supernatant of a human hepatic nonparenchymal cell may contain factors related to pathways listed in FIG. 2-1 to FIG. 2-4 (which are hereinafter sometimes collectively referred to as "specific factors"). It is preferred that the culture supernatant of a human hepatic nonparenchymal cell to be used in the step 1 contain at least one kind, preferably two or more kinds, more preferably all kinds, selected from the specific factors.

In general, the medium 1 is prepared by supplementing a basal medium with the culture supernatant of a human hepatic nonparenchymal cell, the Wnt signaling promoter, and the mitogenic growth factor, and as required, other components to be described later.

Examples of the basal medium to be incorporated into the medium 1 include a BME medium, a BGJb medium, a CMRL 1066 medium, a Glasgow MEM (GMEM) medium, an Improved MEM Zinc Option medium, an IMDM medium, a Medium 199 medium, an Eagle MEM medium, an α-MEM medium, a DMEM medium, an F-12 medium, a DMEM/F12 medium, an IMDM/F12 medium, a Ham medium, an RPMI 1640 medium, and a Fischer's medium, and mixed media thereof.

The culture supernatant of a human hepatic nonparenchymal cell to be incorporated into the medium 1 may be prepared by separating, from a medium after culture of a human hepatic nonparenchymal cell, a supernatant thereof by centrifugation or the like. The method of preparing the culture supernatant of a human hepatic nonparenchymal cell is described in detail later. When the total concentration of the specific factors contained in the culture supernatant of a human hepatic nonparenchymal cell is from 1 ng/mL to 1 mg/mL, the content of the culture supernatant of a human hepatic nonparenchymal cell contained in the medium 1 is generally from 2.5 volume (v/v)% to 50 volume (v/v)%, preferably from 5 volume (v/v)% to 30 volume (v/v)%, more preferably from 10 volume (v/v)% to 20 volume (v/v)% in the total volume of the medium 1.

A Wnt signal functions as a factor in promoting transcription, and mainly controls the protein level of β-catenin, to thereby regulate the growth and differentiation of cells. The Wnt signaling promoter (including a Wnt agonist) is an agonist that activates the Wnt signaling pathway. Examples of the Wnt signaling promoter include a Wnt family member protein, an R-spondin family protein, Norrin, and a glycogen synthase kinase (GSK) inhibitor.

Examples of the Wnt family member protein include Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, and Wnt16. Of those, Wnt3a is preferred. Amino acid sequence information on each of those proteins of the Wnt family may be obtained from an NCBI database ([www.ncbi.nlm.nih.gov/]).

It is known that afamin contributes to the stabilization and solubilization of the Wnt family member protein. Accordingly, the Wnt family member protein is preferably used as a complex with afamin. Afamin refers to a glycoprotein belonging to the albumin family. Examples of afamin include human afamin (GenBank accession number: AAA21612.1) and bovine afamin (GenBank accession number: DAA28569.1).

The Wnt family member protein, or the complex of the Wnt family member protein and afamin may be used as a mature culture solution (conditioned medium) containing the protein or the complex. The concentration of the Wnt family member protein in the conditioned medium is preferably from 18 ng/mL to 900 ng/mL. When the conditioned medium in which the concentration of the Wnt family member protein falls within the above-mentioned range is used as the Wnt family member protein, the content of the conditioned medium in the medium 1 is generally from 1 volume (v/v)% to 50 volume (v/v)%, preferably from 10 volume (v/v)% to 30 volume (v/v)%, more preferably from 15 volume (v/v)% to 25 volume (v/v)% in the total volume of the medium 1.

Examples of the R-spondin family protein include R-spondin 1, R-spondin 2, R-spondin 3, and R-spondin 4. Of those, R-spondin 1 is preferred. Amino acid sequence information on each protein of the R-spondin family may be obtained from an NCBI database. The R-spondin family protein, when bound to Lgr5 in a cell membrane, is removed from the cell membrane through auto-ubiquitinylation, and as a result, Frizzled, which induces the activation of the Wnt signaling pathway, activates the β-catenin pathway in the cell membrane.

The R-spondin family protein may be used as a conditioned medium containing the protein at a concentration of from 0.13 ug/mL to 6.5 ug/mL. When the conditioned medium in which the concentration of the R-spondin family protein falls within the above-mentioned range is used as the R-spondin family protein, the content of the conditioned medium in the medium 1 is generally from 1 volume (v/v)% to 50 volume (v/v)%, preferably from 5 volume (v/v)% to 25 volume (v/v)%, more preferably from 8 volume (v/v)% to 20 volume (v/v)% in the total volume of the medium 1.

The GSK inhibitor refers to an inhibitor of glycogen synthase kinase 3β (GSK3β). GSK3β phosphorylates β-catenin to promote its degradation reaction, to thereby inhibit the Wnt signal, and hence the GSK inhibitor acts as a Wnt signaling promoter. Examples of the GSK inhibitor include CHIR99021 (CAS No.: 252917-06-9), SB216763 (CAS No.: 280744-09-4), SB415286 (CAS No.: 264218-23-7), CHIR98014 (CAS No.: 252935-94-7), AZD1080 (CAS No.: 612487-72-6), and LY2090314 (CAS No.: 603288-22-8).

As the Wnt signaling promoter, a Wnt family protein and an R-spondin family protein are preferably used in combination, Wnt3a and R-spondin-1 are more preferably used in combination, and a complex of Wnt3a and afamin, and R-spondin-1 are still more preferably used in combination.

The mitogenic growth factor initiates signaling involving a mitogen-activated protein kinase (MAPK), and is involved in the growth of cells. Examples of the mitogenic growth factor include an epidermal growth factor (EGF), a fibroblast growth factor (FGF), a hepatocyte growth factor (HGF), an insulin-like growth factor (IGF), and a vascular endothelial growth factor (VEGF). Of those, one or more kinds selected from: an EGF; a FGF; and a HGF are preferred, and a combination of an EGF, a FGF, and a HGF is more preferred. The mitogenic growth factor is preferably a human protein.

The EGF is a growth factor that activates an epidermal growth factor receptor (EGFR or ErbB1). The activated EGFR mainly activates the MAPK signaling pathway, and also activates the PI3K signaling pathway and the Jak/stat signaling pathway. The concentration of the EGF in the medium 1 is generally from 0.5 ng/mL to 1,000 ng/mL, preferably from 1 ng/mL to 500 ng/mL, more preferably from 2 ng/mL to 200 ng/mL.

The FGF is preferably one capable of binding to any one of FGF receptor 1 (FGFR1), FGF receptor 2 (FGFR2), FGF receptor 3 (FGFR3), and FGF receptor 4 (FGFR4), and preferred examples thereof include FGF1, FGF2, FGF3, FGF4, FGF7, and FGF10. In addition, as the FGF, for example, a chimeric product of different kinds of FGFs such as a chimeric FGF including part of regions of FGF1 and part of regions of FGF2 (hereinafter sometimes referred to as "FGFC") may be used. The concentration of the FGF in the medium 1 is generally from 0.5 ng/mL to 1,000 ng/mL, preferably from 1 ng/mL to 500 ng/mL, more preferably from 2 ng/mL to 200 ng/mL.

The HGF is a growth factor that activates a Met receptor, and the activated Met receptor activates the HGF-Met signaling pathway. The activation of the HGF-Met signaling pathway promotes the activation of the β-catenin pathway to promote angiogenesis and metalloprotease production. The concentration of the HGF in the medium 1 is generally from 0.5 ng/mL to 1,000 ng/mL, preferably from 1 ng/mL to 500 ng/mL, more preferably from 2 ng/mL to 200 ng/mL.

The medium 1 may further contain, in addition to the components given above, one or more kinds selected from: a Rho-kinase (ROCK) signaling inhibitor; a transforming growth factor-β (TGF-β) signaling inhibitor; and an interleukin-6 (IL-6) family cytokine.

The medium 1 preferably contains a ROCK signaling inhibitor from the viewpoint of apoptosis suppression. The ROCK signaling inhibitor acts as an antagonist of IGF-1 signaling. Examples of the ROCK signaling inhibitor include Y-27632 (CAS No.: 146986-50-7), fasudil (CAS No.: 105628-07-7), Y39983 (CAS No.: 203911-26-6), WF-536 (CAS No.: 539857-64-2), SLx-2119 (CAS No.: 911417-87-3), azabenzimidazole-aminofurazan (GSK269962) (CAS No.: 850664-21-0), DE-104, H-1152P (CAS No.: 872543-07-6), a Rho kinase α inhibitor (ROKα inhibitor), XD-4000, HMN-1152, 4-(1-aminoalkyl)-N-(4-pyridyl)cyclohexane-carboxamides, Rhostain, BA-210, BA-207, Ki-23095, and VAS-012. Of those, Y-27632 is preferred. The concentration of the ROCK signaling inhibitor in the medium 1 is generally from 1 µM to 20 µM, preferably from 5 µM to 15 µM, more preferably from 8 µM to 12 µM.

The medium 1 preferably contains a TGF-β signaling inhibitor from the viewpoint of the growth properties of the human hepatic parenchymal cell. A TGF-β signal contributes to the suppression of growth of cells, the induction of differentiation and apoptosis of cells, and the like. The TGF-β signaling inhibitor downregulates the TGF-β signal. The TGF-β signaling inhibitor is, for example, an inhibitor that inhibits the activation of a type I receptor or a type II receptor among serine/threonine kinase-type receptors, and examples thereof include: a TGF-β inhibitor that causes the inhibition of phosphorylation of Smad2/3; and a BMP inhibitor that causes the inhibition of phosphorylation of Smad1/5/9.

Examples of the TGF-β inhibitor that causes the inhibition of phosphorylation of Smad2/3 include A83-01 (CAS No.: 909910-43-6), SB-431542 (CAS No.: 301836-41-9), SB-505124 (CAS No.: 694433-59-5), SB-525334 (CAS No.: 356559-20-1), LY364947 (CAS No.: 396129-53-6), SD208 (CAS No.: 627536-09-8), and SJN2511 (CAS No.: 446859-33-2). Of those, A83-01 is preferred. The concentration of the TGF-β inhibitor in the medium 1 is generally from 0.05 µM to 50 µM, preferably from 0.5 µM to 30 µM, more preferably from 1 µM to 15 µM.

Examples of the BMP inhibitor that causes the inhibition of phosphorylation of Smad1/5/9 include noggin, differential screening-selected gene aberrative in neuroblastoma (DAN), and a DAN-like protein. Of those, noggin is preferred. The concentration of the BMP inhibitor in the medium 1 is generally from 10 ng/mL to 100 ng/mL, preferably from 15 ng/mL to 50 ng/mL, more preferably from 20 ng/mL to 30 ng/mL.

The medium 1 preferably contains an IL-6 family cytokine from the viewpoint of the growth properties of the human hepatic parenchymal cell. Being an inflammatory cytokine, the IL-6 family cytokine is produced from Kupffer cells in a living body, and is considered to activate the Kupffer cells themselves or activate MAPK signaling, thereby contributing to the growth of human hepatic parenchymal cells. Examples of the IL-6 family cytokine include interleukin-6 (IL-6), interleukin-11 (IL-11), oncostatin M (OSM), a leukemia inhibitory factor (LIF), cardiotrophin-1 (CT-1), and a ciliary neurotrophic factor (CNTF). Of those, IL-6 is preferred. The concentration of the IL-6 family cytokine in the medium 1 is generally from 10 ng/mL to 1.0 ug/mL, preferably from 50 ng/mL to 500 ng/mL, more preferably from 80 ng/mL to 200 ng/mL.

Further, the medium 1 may contain one or more kinds selected from: retinoic acid; nicotinamide; a cAMP activator; gastrin; a neurobiological supplement; and an antioxidant.

The incorporation of retinoic acid into the medium 1 can enhance the orientation of the human hepatic parenchymal cell. The concentration of retinoic acid in the medium 1 is generally from 1 µM to 30 µM. The incorporation of nicotinamide into the medium 1 can improve cell growth capacity. The concentration of nicotinamide in the medium 1 is generally 5 mM or less. An example of the cAMP activator is forskolin. The incorporation of the cAMP activator into the medium 1 can increase the intracellular concentration of adenosine monophosphate (AMP), and as a result, can reactivate a cell receptor. The concentration of the cAMP activator in the medium 1 is generally from 0.1 µM to 100 µM. The concentration of gastrin in the medium 1 is generally from 5 nM to 15 nM. An example of the antioxidant is N-acetylcysteine. The concentration of the antioxidant in the medium 1 is generally from 0.1 mM to 10 mM. Examples of the neurobiological supplement include insulin-containing supplements, such as a B27 supplement (Thermo Fisher Scientific Inc.) and an N2 supplement (Thermo Fisher Scientific Inc.). The content of the neurobiological supplement in the medium 1 is generally from 0.5 (v/v)% to 10 (v/v)% in the total volume of the medium 1.

### <Method of preparing Culture Supernatant of Human Hepatic Nonparenchymal Cell>

The culture supernatant of a human hepatic nonparenchymal cell to be used in the step 1 is prepared by culturing a human hepatic nonparenchymal cell in a medium for human hepatic nonparenchymal cell culture containing a Wnt signaling promoter and a mitogenic growth factor (hereinafter sometimes referred to as "medium 2"), and separating a supernatant from the medium after the culture. Examples of the human hepatic nonparenchymal cell to be subjected to the culture include a hepatic stellate cell, a sinusoidal endothelial cell, a Kupffer cell, and a mixture of two or more kinds thereof.

In general, the medium 2 may be prepared by supplementing a basal medium with the Wnt signaling promoter and the mitogenic growth factor, and as required, other components to be described later. Examples of the basal medium contained in the medium 2 include the same ones as those of the basal medium for the medium 1.

The medium 2 preferably contains the Wnt signaling promoter and the mitogenic growth factor. Examples of the Wnt signaling promoter and the mitogenic growth factor that may be used for the medium 2, and the contents thereof in the medium 2 are as exemplified above for the medium 1.

The medium 2 preferably further contains a ROCK signaling inhibitor in addition to the components given above. Examples of the ROCK signaling inhibitor that may be used for the medium 2 and the concentration thereof in the medium 2 are as exemplified above for the medium 1. As required, the medium 2 may contain, as the other component, one or more kinds selected from: an IL-6 family cytokine; a TGF-β signaling inhibitor; retinoic acid; nicotinamide; a cAMP activator such as forskolin; gastrin; a neurobiological supplement; and an antioxidant such as N-acetylcysteine, in addition to the above-mentioned components. Examples of the other component that may be used for the medium 2 and the concentration thereof in the medium 2 are as exemplified above for the medium 1.

A method for the culture of the human hepatic nonparenchymal cell with the medium 2 is preferably suspension culture. At the time of the culture, the human hepatic nonparenchymal cell is preferably cultured while being brought into contact with an ECM. The human hepatic nonparenchymal cell may be brought into contact with the ECM by, for example, embedding the human hepatic nonparenchymal cell in the ECM, or adding the human hepatic nonparenchymal cell to a medium having the ECM suspended therein. In a preferred embodiment, in accordance with a conventional method, an ECM precursor is brought into contact with the human hepatic nonparenchymal cell in the process of being gelled, to thereby prepare a human hepatic nonparenchymal cell embedded in the ECM. Examples of the ECM or the ECM precursor to be used in the culture of the human hepatic nonparenchymal cell include those given as examples above for the step 1 such as Matrigel (trademark).

The culture of the human hepatic nonparenchymal cell is performed under the temperature condition of generally from 30°C to 50°C, preferably from 32°C to 48°C, more preferably from 34°C to 46°C. In addition, the culture of the human hepatic nonparenchymal cell is performed under an atmosphere having a carbon dioxide content of generally from 1 vol% to 15 vol%, preferably from 2 vol% to 14 vol%, more preferably from 3 vol% to 13 vol%. The culture is performed for a period of generally 1 day or more, preferably from 1 day to 20 days.

The culture supernatant of a human hepatic nonparenchymal cell to be used in the step 1 may be prepared by recovering a supernatant, by a general procedure such as centrifugation, from the medium after the culture of the human hepatic nonparenchymal cell thus obtained.

It is preferred that the prepared culture supernatant of a human hepatic nonparenchymal cell contain at least one or more kinds, preferably two or more kinds, more preferably all kinds of the specific factors related to the genes shown in FIG. 2-1 to FIG. 2-4.

The total concentration of the specific factors contained in the culture supernatant of a human hepatic nonparenchymal cell is generally from 1 ng/mL to 1 mg/mL, preferably from 10 ng/mL to 100 µg/mL.

### <Cultured Human Hepatic Parenchymal Cell>

According to another embodiment of the present invention, there is provided a cultured human hepatic parenchymal cell obtained by the above-mentioned method of culturing a human hepatic parenchymal cell.

The cultured human hepatic parenchymal cell provided in this embodiment is a human hepatic parenchymal cell capable of growing. The human hepatic parenchymal cell according to this embodiment has drug-metabolizing activity. The drug-metabolizing activity of the cell may be evaluated through detection of the expression of a drug-metabolizing enzyme or drug metabolism assay. Examples of the drug-metabolizing enzyme that may be expressed by the human hepatic parenchymal cell according to this embodiment include cytochrome P450 1A2 (CYP1A2), cytochrome P450 2B (CYP2B), cytochrome P450 2C9 (CYP2C9), cytochrome P450 2C19 (CYP2C19), cytochrome P450 2D6 (CYP2D6), cytochrome P450 2E1 (CYP2E1), cytochrome P450 3A4 (CYP3A4), cytochrome P450 3A7 (CYP3A7), a uridine diphosphate-glucuronosyltransferase (UGT), and a sulfotransferase (SULT).

It is preferred that the drug-metabolizing activity of the cultured human hepatic parenchymal cell according to this embodiment be generally as follows when the expression amount of the drug-metabolizing enzyme of the human hepatic parenchymal cell before culture is defined as 100: from 20 to 200 for CYP1A2; from 10 to 200 for CYP2B; from 20 to 200 for CYP2C9; from 20 to 200 for CYP2C19; from 10 to 190 for CYP2D6; from 1 to 150 for CYP2E1; from 0.1 to 200 for CYP3A4; from 0.1 to 120 for YP3A7; from 10 to 300 for UGT; or from 20 to 250 for SULT.

When the cultured human hepatic parenchymal cell according to this embodiment is subjected to differentiation culture, there can be obtained a mature human hepatic parenchymal cell having a further improved expression amount of the drug-metabolizing enzyme. As a method for the differentiation culture, there is given, for example, a culture method involving regulating TGF-β signaling, Wnt signaling, and Notch signaling, which contribute to the growth of human hepatic parenchymal cells. A preferred example thereof is a method involving culturing the human hepatic parenchymal cell in a medium containing (3,5-difluorophenylacetyl)-L-alanyl-L-2-phenylglycine tert-butyl (DAPT; CAS No.: 208255-80-5). Differentiation into a mature human hepatic parenchymal cell may be recognized by performing gene expression analysis of the cell.

### Examples

Now, the present invention is described in more detail by way of experimental examples. However, the present invention is by no means limited by these experimental examples. In the following experimental examples, sterilized instruments were used for all experiments. In addition, all experiments were performed on the basis of an ethical research plan approved by the Keio University School of Medicine Ethics Committee.

### [Media]

Media A to E shown in Table 1 were prepared. Advanced DMEM/F12 was used as a basal medium. In each of the media, R-spondin-1 was used in the form of a conditioned medium containing R-spondin-1, and the concentration of R-spondin-1 with respect to the total volume of the conditioned medium was 1.3 ug/mL. In addition, in each of the media, Wnt3a was used in the form of a conditioned medium containing a complex of Wnt3a and afamin, and the concentration of Wnt3a with respect to the total volume of the conditioned medium was 360 ng/mL. The media B and C and F were each supplemented with a culture supernatant of human hepatic nonparenchymal cells prepared in Experimental Example 1 to be described later.

**Table 1**

| Advanced DMEM/F12 with: | Medium A | Medium B | Medium C | Medium D | Medium E | Medium F |
|---|---|---|---|---|---|---|
| B27 supplement | 2% (v/v) | 2% (v/v) | 2% (v/v) | 2% (v/v) | 2% (v/v) | 2% (v/v) |
| N2 supplement | 1% (v/v) | 1% (v/v) | 1% (v/v) | 1% (v/v) | 1% (v/v) | 1% (v/v) |
| N-acetylcysteine | 1 mM | 1 mM | 1 mM | 1 mM | 1 mM | 1 mM |
| R-spondin-1 conditioned medium | 10% (v/v) | 10% (v/v) | 10% (v/v) | 10% (v/v) | 10% (v/v) | 10% (v/v) |
| Wnt3a/afamin-conditioned medium | 200 (v/v) | 20% (v/v) | 20% (v/v) | 20% (v/v) | 20% (v/v) | 20% (v/v) |
| Noggin | 25 ng/mL | 25 ng/mL | 25 ng/mL | 25 ng/mL | 25 ng/mL | 25 ng/mL |
| (Leu15)-gastrin I | 10 nM | 10 nM | 10 nM | 10 nM | 10 nM | 10 nM |
| EGF | 4 ng/mL | 4 ng/mL | 4 ng/mL | 4 ng/mL | 4 ng/mL | 4 ng/mL |
| FGF10 | 100 ng/mL | 100 ng/mL | 100 ng/mL | 100 ng/mL | 100 ng/mL | 100 ng/mL |
| HGF | 50 ng/mL | 50 ng/mL | 50 ng/mL | 50 ng/mL | 50 ng/mL | 50 ng/mL |
| Y27632 | 10 µm | 10 µm | 10 µm | 10 µm | 10 µm | 10 um |
| Forskolin | 10 µm | 10 µm | 10 µm | 10 µm | 10 µm | 10 µm |
| A83-01 | - | 5 µm | - | 5 µm | 5 µm | 5 um |
| IL-6 | 100 ng/mL | 100 ng/mL | 100 ng/mL | 100 ng/mL | 100 ng/mL | - |
| DAPT (CAS No.: 208255-80-5) | - | - | - | - | 10 µm | - |
| Culture supernatant of human hepatic nonparenchymal cells of Experimental Example 1 | - | 20% (v/v) | 20% (v/v) | - | - | 20% (v/v) |

### [Experimental Example 1] Preparation of Culture Supernatant of Human Hepatic Nonparenchymal Cells

Frozen primary human hepatic nonparenchymal cells ("NPC-101", BIOREWDIC) were thawed in a water bath at 37°C, and the cells were suspended in a 50 mL tube having added thereto a serum-free medium and were centrifuged. The serum-free medium was prepared by adding HEPES, GlutaMAX, and penicillin/streptomycin to Advanced DMEM/F12. After the centrifugation, the supernatant was removed, and then the cells were suspended in the serum-free medium to prepare a suspension of human hepatic nonparenchymal cells. 10,000 human hepatic nonparenchymal cells were taken out of the suspension, mixed with 50 µL of Matrigel (trademark) (BD Biosciences), seeded in each well of a 24-well tissue culture plate, and incubated at 37°C for from 5 minutes to 10 minutes until the Matrigel fully polymerized. After the Matrigel had polymerized, a medium containing the components shown in "Medium A" of Table 1 was overlaid thereon, and the human hepatic nonparenchymal cells were cultured for 5 days. The medium was centrifuged, and then the culture supernatant of the human hepatic nonparenchymal cells was recovered.

### [Experimental Example 2] Culture of Human Hepatic Parenchymal Cells

Frozen primary human hepatic parenchymal cells ("HEP187-S", BIOPRRIDIC, containing a trace amount of human cholangiocytes) were thawed in a water bath at 37°C, and the cells were suspended in a 50 mL tube having added thereto a serum-free medium and were centrifuged. The serum-free medium was prepared by adding HEPES, GlutaMAX, and penicillin/streptomycin to Advanced DMEM/F12. After the centrifugation, the supernatant was removed, and then the cells were suspended in the serum-free medium to prepare a suspension of human hepatocytes (containing the human cholangiocytes). 10,000 cells were taken out of the suspension, mixed with 50 µL of Matrigel (trademark) (BD Biosciences), seeded in each well of a 24-well tissue culture plate, and incubated at 37°C for from 5 minutes to 10 minutes until the Matrigel fully polymerized. After the Matrigel had polymerized, a medium containing the components shown in "Medium B" of Table 1 was overlaid thereon, and the human hepatic parenchymal cells were cultured while the medium was changed every 5 days. Periodically, the culture product was collected, the cells were recovered by centrifugation, and the number of cells was counted. The number of cells increased from the start of the culture was calculated to evaluate the growth properties of the cells.

### [Experimental Example 3] Culture of Human Hepatic Parenchymal Cells

Human hepatic parenchymal cells were cultured by the same operations as in Experimental Example 2 except that the medium C shown in Table 1 was used in place of the medium B. The cells were periodically recovered and counted to evaluate their growth properties in the same manner as in Experimental Example 2.

### [Experimental Example 4] Culture of Human Hepatic Parenchymal Cells

Human hepatic parenchymal cells were cultured by the same operations as in Experimental Example 2 except that the medium D shown in Table 1 was used in place of the medium B. The cells were periodically recovered and counted to evaluate their growth properties in the same manner as in Experimental Example 2.

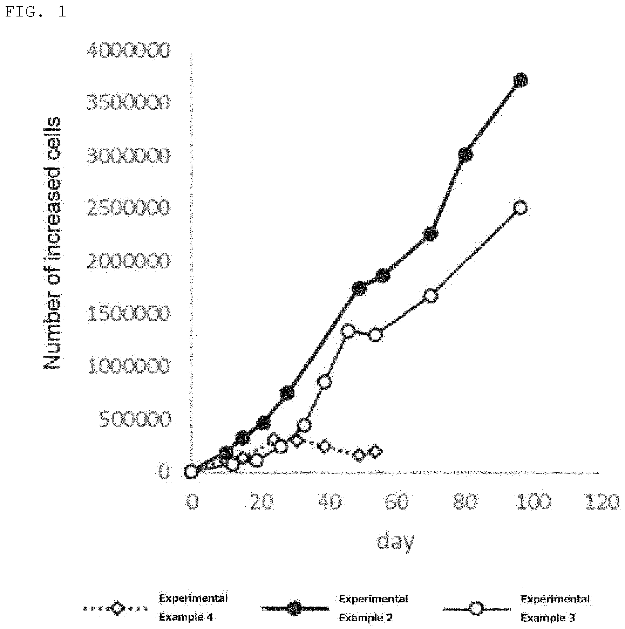

The numbers of increased cells in Experimental Example 2 to Experimental Example 4 are shown in FIG. 1.

### [Experimental Example 5] Gene Expression Analysis of Cultured Human Hepatic Parenchymal Cells

Through RNA-seq analysis of the human hepatic parenchymal cells of Experimental Example 2 and Experimental Example 4 after 2 weeks of culture, the expression amounts of various genes in the cells of Experimental Example 2 and Experimental Example 4 were measured. On the basis of the gene expressions analyzed by RNA-seq, pathways that were likely activated were estimated using pathway analysis software IPA. The results are shown in FIG. 2-1 to FIG. 2-4. In FIG. 2-1 to FIG. 2-4, NPC(-) represents the human hepatic parenchymal cells of Experimental Example 4, and NPC(+) represents the human hepatic parenchymal cells of Experimental Example 2. There were pathways having different activation states between NPC(-) and NPC(+). It is possible that factors related to those activated pathways contribute to the growth of hepatic parenchymal cells.

### [Experimental Example 6] Mature Human Hepatic Parenchymal Cells

The cultured human hepatic parenchymal cells of Experimental Example 2 after 2 weeks of culture were subjected to dispersion treatment by mechanical dissociation to prepare a suspension of human hepatic parenchymal cells. 10,000 cells were taken out of the suspension, mixed with 50 µL of Matrigel (trademark) (BD Biosciences), seeded in each well of a 24-well tissue culture plate, and incubated at 37°C for from 5 minutes to 10 minutes until the Matrigel fully polymerized. After the Matrigel had polymerized, a medium containing the components shown in "Medium E" of Table 1 was overlaid thereon, and the cultured human hepatic parenchymal cells of Experimental Example 2 were cultured while the medium was changed every 7 days. The resultant culture product was centrifuged, and the cells were recovered.

### [Experimental Example 7] Culture of Human Cholangiocytes

Frozen primary human hepatic parenchymal cells ("HEP187-S", BIOPRRIDIC, containing a trace amount of human cholangiocytes) were thawed in a water bath at 37°C, and the cells were suspended in a 50 mL tube having added thereto a serum-free medium and were centrifuged. The serum-free medium was prepared by adding HEPES, GlutaMAX, and penicillin/streptomycin to Advanced DMEM/F12. After the centrifugation, the supernatant was removed, and then the cells were suspended in the serum-free medium to prepare a suspension of human hepatic parenchymal cells (containing the human cholangiocytes). 10,000 cells were taken out of the suspension, mixed with 50 µL of Matrigel (trademark) (BD Biosciences), seeded in each well of a 24-well tissue culture plate, and incubated at 37°C for from 5 minutes to 10 minutes until the Matrigel fully polymerized. After the Matrigel had polymerized, a medium containing the components shown in "Medium B" of Table 1 was overlaid thereon, and the human cholangiocytes were cultured while the medium was changed every 5 days and while the grown human hepatic parenchymal cells were removed from the medium as appropriate.

### [Experimental Example 8] Comparative Analysis of Gene Expressions

Gene expression analysis by RNA-seq was performed for primary human hepatic parenchymal cells serving as a control ("HEP187-S", BIOPRRIDIC, hereinafter referred to as "PHHs"), the cultured human hepatic parenchymal cells of Experimental Example 2 after 10 days of culture (hereinafter referred to as "Proli-Orgs"), the mature human hepatic parenchymal cells after 10 days of culture in Experimental Example 6 (hereinafter referred to as "Differ-Orgs"), the mature human hepatic parenchymal cells after 100 days of culture in Experimental Example 6 (hereinafter referred to as "Differ-Orgs (long-term)"), and the human cholangiocytes of Experimental Example 7 after 30 days of culture (hereinafter referred to as "Chol").

The expression amounts of 57,095 kinds of genes, such as metabolizing enzymes, transporter genes, and hepatocyte markers, were measured by RNA-seq analysis. Read count data obtained by RNA-seq was corrected with a total read count and then corrected by transcript length (FRKM).

FIG. 3 shows correlation coefficients for linear regression of the gene expression amounts obtained by the RNA-seq analysis among the PHHs, the Proli-Orgs, the Differ-Orgs, the Differ-Orgs (long-term), and the Chol. As shown in FIG. 3, the Proli-Orgs of Experimental Example 2, and the Differ-Orgs and Differ-Orgs (long-term) of Experimental Example 6 each had a gene expression pattern showing a relatively high correlation with that of the PHHs, and not being similar to that of the Chol. Thus, it was shown that those cells had not transformed into human hepatic nonparenchymal cells or other cells other than hepatic parenchymal cells.

FIG. 4 shows microscopic images and gene expression amounts of the Proli-Orgs and the Differ-Orgs (long-term). In FIG. 4, the gene expression amounts represent relative expression amounts with each of the expression amounts of the PHHs being defined as +++, and a larger number of "+"s indicates a higher gene expression amount. The microscopic images revealed that a component of the lumen of the Proli-Orgs was red, and a component of the lumen of the Differ-Orgs was yellow, indicating that the lumen contained bile. The results of FIG. 3 and FIG. 4 showed that the Differ-Orgs (long-term) of Example 3 had a high similarity of gene expression pattern to the PHHs, thus being usable for pharmacokinetic tests for pharmaceutical development and the like. Those results also show that the cultured human hepatic parenchymal cells of Experimental Example 2 retain an ability to differentiate into mature human hepatic parenchymal cells.

### [Experimental Example 9]

Human hepatic parenchymal cells were cultured by the same operations as in Experimental Example 2 except that the medium F shown in Table 1 was used in place of the medium B. The growth properties of the cells were recognized with microscopic images. Microscopic images of the cultured human hepatic parenchymal cells of Experimental Example 2 and Experimental Example 9 are shown in FIG. 5-1 and FIG. 5-2, respectively.

### [Experimental Example 10]

Human hepatic parenchymal cells were cultured by the same operations as in Experimental Example 2 except that a collagen-coated plate was used in place of Matrigel embedding. A microscopic image is shown in FIG. 6. It was recognized from the result of Experimental Example 10 that the human hepatic parenchymal cells grew two-dimensionally in the collagen-coated plate.

## Claims

1. A method of culturing a human hepatic parenchymal cell, comprising culturing a human hepatic parenchymal cell in a medium containing:
a culture supernatant of a human hepatic nonparenchymal cell;
a Wnt signaling promoter; and
a mitogenic growth factor.

2. The method according to claim 1, wherein the Wnt signaling promoter contains a Wnt family member protein.

3. The method according to claim 2, wherein the Wnt signaling promoter further contains an R-spondin super family protein.

4. The method according to any one of claims 1 to 3, wherein the mitogenic growth factor contains one or more kinds selected from the group consisting of: an epidermal growth factor (EGF); a fibroblast growth factor (FGF); and a hepatocyte growth factor (HGF).

5. The method according to any one of claims 1 to 4, wherein the medium further contains one or more kinds selected from: a Rho-kinase (ROCK) signaling inhibitor; a transforming growth factor-β (TGF-β) signaling inhibitor; and an interleukin-6 (IL-6) family cytokine.

6. The method according to claim 5, wherein the medium contains the ROCK signaling inhibitor.

7. The method according to claim 5 or 6, wherein the medium contains the TGF-β signaling inhibitor.

8. The method according to any one of claims 5 to 7, wherein the medium contains the IL-6 family cytokine.

9. The method according to any one of claims 1 to 8, wherein the medium further contains one or more kinds selected from: retinoic acid; nicotinamide; a cAMP activator; gastrin; a neurobiological supplement; and an antioxidant.

10. The method according to any one of claims 1 to 9, wherein the culture supernatant of a human hepatic nonparenchymal cell is produced by:
culturing a human hepatic nonparenchymal cell in a medium for human hepatic nonparenchymal cell culture, the medium containing a Wnt signaling promoter and a mitogenic growth factor; and
separating a supernatant from the medium after the culturing.

11. The method according to claim 10, wherein the medium for human hepatic nonparenchymal cell culture further contains a ROCK signaling inhibitor.

12. The method according to claim 10 or 11, wherein the medium for human hepatic nonparenchymal cell culture further contains one or more kinds selected from: an IL-6 family cytokine; a TGF-β signaling inhibitor; retinoic acid; nicotinamide; a cAMP activator; gastrin; a neurobiological supplement; and an antioxidant.

13. A cultured human hepatic parenchymal cell, which has been cultured by the method of any one of claims 1 to 12.

14. A medium, comprising:
a culture supernatant of a human hepatic nonparenchymal cell;
a Wnt signaling promoter; and
a mitogenic growth factor.

15. A method of producing a culture supernatant of a human hepatic nonparenchymal cell, the method comprising:
culturing a human hepatic nonparenchymal cell in a medium containing a Wnt signaling promoter and a mitogenic growth factor; and
separating a supernatant from the medium after the culturing.

16. A method of producing a medium, comprising mixing a culture supernatant of a human hepatic nonparenchymal cell produced by the method of claim 15, a Wnt signaling promoter, and a mitogenic growth factor.
